Europäisches Patentamt

European Patent Office

Office européen des brevets

(19)

(11) Publication number: **0 235 563**
**B1**

(12)

# EUROPEAN PATENT SPECIFICATION

(45) Date of publication of the patent specification:
24.10.90

(51) Int. Cl.⁵: **A61F 5/448**

(21) Application number: 87101088.0

(22) Date of filing: 24.06.83

(60) Publication number of the earlier application in accordance
with Art. 76 EPC: 0098718

(54) Improved ostomy device.

(30) Priority: 24.06.82 US 391577

(43) Date of publication of application:
09.09.87 Bulletin 87/37

(45) Publication of the grant of the patent:
24.10.90 Bulletin 90/43

(84) Designated Contracting States:
AT BE CH DE FR GB IT LI LU NL SE

(56) References cited:
GB-A- 1 571 657
GB-A- 1 583 027
US-A- 4 078 567

(73) Proprietor: E.R. Squibb & Sons, Inc.,
Lawrenceville-Princeton Road, Princeton,
N.J. 08540-4000(US)

(72) Inventor: Jensen, Ole Roger, 646 Orangeburg Road,
River Vale New Jersey(US)

(74) Representative: Cook, Anthony John et al, D. YOUNG &
CO. 10, Staple Inn, London, WC1V 7RD(GB)

ACTORUM AG

## Description

The present invention relates to ostomy devices of the type including releaseable interengaging parts adapted to permit detachment of the pouch from the adhesive-backed label and, more particularly, to the structure of the means utilized to mount one of the parts to the label.

Subsequent to ileostomy, colostomy and similar surgical procedures, it is often necessary for the patient to utilize an ostomy pouch to cover the stoma and collect waste mateial as it is discharged. Over the years, ostomy pouches of a variety of different sizes, shapes and constructions have been utilized. Various materials and adhesives have been developed to increase the utility and wearability of the pouches.

The basic pouch comprises first and second thin film walls which are sealed, by heat welding or the like, along their periphery to form the contour of the pouch. One wall has an aperture therein designed to receive the stoma. Affixed to the exterior surface of the aperture bearing wall is an adhesive-backed flange or label designed to secure the pouch to the skin surrounding the stoma. The adhesive-backed label has an aperture which aligns with the aperture in the pouch wall. The adhesive-backed label may be affixed to the exterior surface of the pouch in any suitable manner, such as by welding or laminating the adhesive-backed label to the pouch wall. The weld or lamination may take the form of a ring surrounding the aligned apertures.

Over the years, significant advances have been made in the materials employed to construct the labels, particularly in the composition of the base and adhesive coating, such that the labels have become increasingly flexible and porous, permitting the labels to be comfortably worn for extended periods of time. Recent improvements have extended the period during which the labels can be comfortably worn to be longer than normally required for the pouch to fill to capacity with waste material. However, it is still necessary, particularly with new users, to frequently remove the label from the skin to provide access to the skin surrounding the stoma. Removal of the label permits the user to observe and check the condition of the skin surrounding the stoma and, if necessary, to treat same. Thus, in spite of the improvements in label material, frequent removal of the one-piece type device, due to cleaning of the pouch and checking or treatment of the skin surrounding the stoma, is still normally required.

On the other hand, frequent removal of the adhesive-backed label from the skin is to be avoided. The skin surrounding the stoma is often extremely sensitive and may comprise a healing incision or scar tissue. Although the adhesives utilized in the label are formulated to reduce, as much as possible, irritation of the skin when the label is removed, it is preferable to limit the number of times the label must be removed from the skin as much as possible.

One solution to this problem, see patent specification GB-A-1,571,657, is to provide a two-piece ostomy device, wherein the pouch is releasably connectable to the label. The label can then remain affixed to the skin for an extending period of time. The necessity for removing the label each time the pouch is filled is eliminated because the filled pouch can be detached from the label and replaced with a new pouch as often as necessary.

The two-part structure requires a means for releasably connecting the pouch and the label. One particularly successful device takes the form of a pair of annular or ring-like rigid plastic parts - one in the form of a flange, and the other in the form of a groove. The parts are designed to frictionally engage to secure the pouch to the label and, when necessary, to disengage to permit removal of the pouch from the label. The parts are mountd to the pouch and label, respectively. The pouch and label can be connected by simply aligning the parts and pressing same together to cause frictional engagement.

The interengaging part mounted to the pouch surrounds the stoma receiving aperture in the pouch. The part mounted to the label surrounds the aperture in the label. When the interengaging parts are engaged, the apertures automatically correctly align.

When the label is mounted to the skin, the parts are engaged by applying a force on the exterior portion of the pouch over the parts in the direction of the label. This force is of a significant magnitude and is absorbed by the skin beneath the label. However, since the skin under the label may be sensitive, particularly in the time period following the surgical procedure creating the stoma, applying sufficient force to the skin to cause engagement of the parts is often painful and may even be destructive. It is therefore desirable to develop a system which would permit engagement of the parts without the application of significant force on the skin.

It is not possible to simply reduce the amount of force necessary to cause frictional engagement of the parts. This would result in a reduction of the force necessary to detach the pouch from the label, thereby increasing the possibility of accidental detachment. Thus, a different approach to this problem is required.

In patent specification GB-A-2,115,288, published after the present priority date, a modification of the arrangement disclosed in GB-A-1,571,657 is disclosed in which the mounting structure, instead of rigidly retaining the part against the label surface as in GB-A-1,571,657, is made somewhat flexible to permit the user to temporarily lodge his fingers between the part and the surface of the label as the pouch is attached. In this manner, the force applied to interengage the parts is absorbed by the fingers, instead of the sensitive skin surrounding the stoma.

Accordingly, the rigid method of mounting the part of the surface of the label, namely bonding the bottom surface of the part directly to the surface of the label, had to be discarded. Such a structure would not provide the necessary flexibility to permit the fingers to be placed behind the part, when the pouch and label were being connected.

Instead of affixing the bottom surface of the part directly to the label in a rigid fashion, the bottom surface of the flange, which extends outwardly of the part, was affixed to the periphery of a circular film member. The member which extends inwardly from the flange toward the center of the part is provided with a central aperture designed to align with the aperture in the label. A ring-like section of the film member, adjacent the aperture, is adapted to be affixed to the surface of the label by welding, lamination, or adhesive. In this manner, the flexibility of the label is somewhat increased and a small space between the part and the surface of the label is provided to permit insertion of the fingers, as the pouch is connected to the label, so as to permit positioning of the fingers to absorb the applied force.

The above-described mounting structure was intended to solve the problem of the application of force to the sensitive skin under the label during the attachment of the pouch. However, other problems inherent in this structure developed. The first problem relates to stress applied to the mounting structure as the fingers are inserted between the part and the surface of the label and to the resulting physical distortion of the interengaging part which makes engagement with the corresponding part difficult. The space available between the part and the surface of the label to accommodate the fingers, even when the surface of the label is flexed, is quite small. For this reason, lodging of the fingers between the part and the surface of the label creates stress on the section of the film member affixed to the label surface at points thereon adjacent the locations where the fingers are inserted. Such stress points tend to dislodge the part from the label because the bond on the section of the film member affixed to the label surface tends to release at the stress points. This structure also results in the lifting of the label from the skin as well as a distortion of the part when the fingers are inserted behind the part.

Another disadvantage of the mounting structure described above relates to the collection of waste material in the space between the surface of the film member and the bottom of the part. Since the film member is bonded to the bottom of the flange which extends outwardly of the part, a pocket is formed between the surface of the film member and the bottom surface of the part. This pocket opens into the interior of the device, toward the aligned stoma receiving openings. Accordingly, waste material moving laterally along the surface of the film member will collect within this pocket where it becomes extremely difficult to remove and provides an area for bacterial growth close to the stoma.

A further disadvantage of the conventional mounting structure is that the pouch surface may be in direct contact with the stoma. While this is normally acceptable, in certain instances, for example, immediately after surgery, it may be desirable to retain the pouch surface at a position spaced from the wound. This is, however, not possible with the structure described above.

Another drawback of conventional detachable pouches, in general, relates to the possibility of the pouch accidentally detaching as it is worn. This is a difficult result to avoid since the amount of force necessary to detach the interengaging parts is determined by the structure thereof and is a function of the amount of force necessary to frictionally engage the parts, which, in turn, is limited, as indicated above, by the amount of force which can be acceptably applied to the skin under the label. Consequently, it is not feasible to alter the structure of the interengaging parts to provide additional security against accidental detachment, unless some complex and costly release mechanism is incorporated into the structure.

Conventional two-piece ostomy devices employ circular interengaging parts. However, there are certain instances where the wound is elongated or the surgical procedure requires the temporary insertion of a plastic tube or glass rod under a section of the intestine. In such circumstances, the circular shape of the interengaging parts may be too restrictive and prevent the use of the device.

In accordance with the present invention, an ostomy device is provided comprising a pouch having a stoma receiving aperture, a separate adhesively-backed label for attachment of the device to the skin of the wearer and having an aperture coaxially aligned with the aperture in the pouch for the passage of the stoma through the label, and annular mounting means for releasably connecting the pouch to the label, wherein the annular mounting means comprise releasably interengaging first and second annular parts each provided with releasably engaging interconnecting means, and means for mounting said first and second parts respectively on the pouch and the label around their respective apertures, characterised in that the first annular part of the mounting means connected to the pouch has a rover having a central aperture therein aligned with the aperture in the pouch and the aperture in the label, said cover extending inwardly from the periphery of said part and axially towards said second part, and having a rim, which, when the two interconnecting parts are engaged, sealingly engages with the surface of the label interiorly of said second part and around the aperture.

In a preferred embodiment of the invention, the second part of the mounting means comprises an annular member having means for releasably engaging with cooperating means on the first part, and wherein the label comprises a flexible backing member coated with a layer of adhesive, said annular member being mounted on the label by a flexible member connected to and extending inward of the annular member and connected to the backing member adjacent the aperture therein, said flexible member permitting the annular member to be drawn away from the label whilst the connection is made between the two interengaging parts.

The said flexible member may include or comprise expandable means.

The first interengaging part preferably comprises a ring-shaped or oval engaging part. The cover means preferably comprises a flange-like element extending from the inner surface of the engaging part towards the center thereof.

In accordance with another aspect of the present invention, means may additionally be provided for retaining the expandable means in the expanded condition.

Retaining the expandable means in the expanded condition maintains the pouch surface at a location spaced from the wound. Preferably, the retaining means takes the form of a detachable ring formed of elastic material to permit same to be inserted underneath the member, preferably that portion of the member in alignment with the connecting means mounted thereon.

In accordance with another aspect of the present invention, the interengageable parts may be elongated, preferably oval shaped, rather than circular.

In accordance with another aspect of the present invention, secondary means may be provided for releasably connecting the pouch and the label in addition to the primary connecting means.

The secondary connecting means preferably comprises a protrusion and aperture structure or a hook and eye structure. The primary connecting means preferably comprise a tongue and groove structure and, more particularly, a ring-shaped flange and a ring-shaped groove.

The secondary connecting means is preferably located proximate the top of the pouch primary connecting means and the label. In this manner, should the primary connecting means become accidentally detached, the secondary connecting means will prevent the pouch from detaching from the label. The secondary connecting means also serves to aid in supporting the pouch as the pouch becomes heavier due to the accumulation of waste material therein.

To these and other such objects which may hereinafter appear, the present invention relates to an improved ostomy device, as set forth in detail int eh following specification and recited in the annexed claims, taken together with the acocmpanying drawings, wherein like numerals refer to like parts, and in which:

Figure 1 is an exploded isometric view of an ostomy device of the type disclosed in GB-A-1,571,657 including a label, a pouch, and means for releasably connecting the label and pouch.

Figure 2 is a cross-sectional view of an ostomy device similar to that shown in Figure 1, but modified in accordance with the proposal disclosed in the aforesaid GB-A-2,115,288;

Figure 3 is an isometric view of a first preferred embodiment of the label portion of an improved ostomy device according to the present invention;

Figure 4 is a cross-sectional view of a first preferred embodiment of an ostomy device according to the present invention;

Figure 5 is a cross-sectional view of the embodiment of Figure 4 illustrating the manner in which the parts are engaged;

Figure 6 is a cross-sectional view of a second embodiment according to the present invention showing the optional expansion retaining means;

Figure 7 is a sectional view taken on lone 15-15 of Figure 6;

Figure 8 is a cross-sectional view of a device according to the present invention illustrating a second preferred embodiment of the expansion retaining means;

Figure 9 is a cross-sectional view of a device according to the present invention illustrating a third preferred embodiment of the expansion retaining means;

Figure 10 is an isometric view showing a first preferred embodiment of the auxiliary connecting parts which may be used in accordance with the present invention;

Figure 11 is an isometric view showing a second preferred embodiment of the auxiliary connecting parts which may be used in accordance with the present invention; and

Figure 12 is a front view of an embodiment according to the present invention; wherein the interengaging parts are oval.

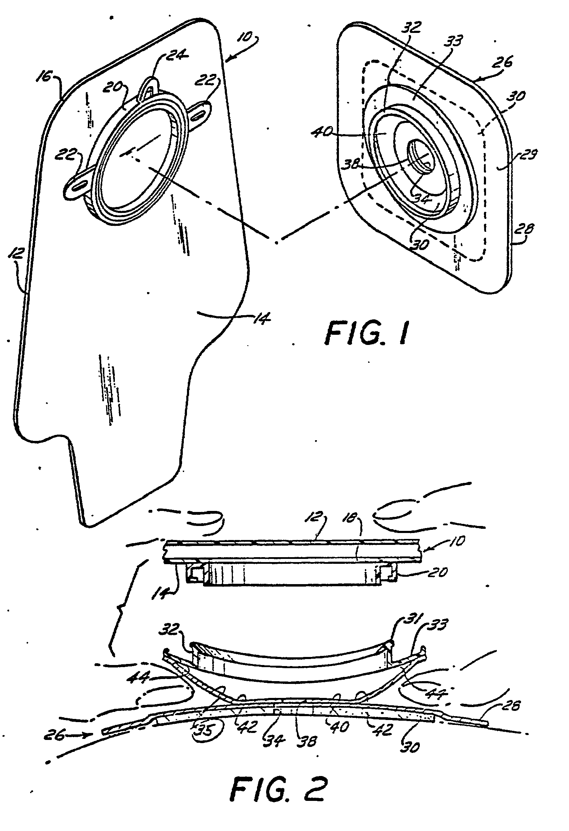

Figure 1 illustrates a commercially available ostomy device of the type shown in GB-A-1,571,657 with a detachable pouch. The pouch generally designated 10, comprises a first or outer wall 12 and a second or inner wall 14, both composed of a thin, moisture-impermeable, odor-proof, thermo-plastic film or laminate. The interior surfaces of walls 12 and 14 are sealed along the periphery 16 thereof, so as to form the contour of the pouch. Wall 14 is provided with a stoma receiving aperture 18.

Surrounding aperture 18 is an annular plastic part 20, preferably in the form of a ring-shaped groove, which forms one of the interconnecting parts. The rear surface of part 20 is affixed to the exterior surface of wall 14 in any appropriate manner, such as welding or by a layer of adhesive or the like. Extending outwardly from the side of part 20, in opposite directions, are a pair of belt-receiving elements 22 which can be utilized to connect part 20 and, thus, pouch 10 to a belt designed to extend around the body. Also extending from the side of part 20, near the top thereof, is a tab 24 designed to facilitate grasping of part 20.

The conventional ostomy device illustrated in Fig. 1 also includes an adhesive-backed label, generally designated 26. The purpose of the adhesive-backed label 26 is to affix the device to the skin of the wearer. The adhesive-backed label 26 must serve to securely retain the device to the skin surrounding the stoma for an extended period of time.

Adhesive-backed label 26 comprises a base 28 which can be formed of porous material and an adhesive layer 30 situated on the rear surface of base 28.

To the front surface 29 of base 28 is mounted the second interengaging part 32, which preferably takes the form of an annular or ring-like protrusion 31 and an outwardly extending flange 33. Protrusion 31 is fashioned to frictionally engage groove-like part 20. Part 32 is mounted on label 26 at a point surrounding an aperture 34 in the label. Aperture 34 in label 26 is designated to align with aperture 18 in pouch 10 when parts 20 and 32 are frictionally engaged.

As discussed previously, part 32 could be affixed directly to surface 29 of base 28. However, this does not permit the user to insert his fingers between part 32 and label 26, so as to absorb the force applied to interengage parts 20 and 32.

Consequently, a modified mounting method may be used, here shown in Figure 2, this modification being of the type disclosed in the aforesaid patent specification GB-A-2,115,288. In this case, part 32 is mounted to base 28 of label 26 through the use of a circular-shaped thin film member 35. Film 35 is provided with an aperture 38, at the central portion thereof. Aperture 38 aligns with aperture 34 in label 26. Film 35 is affixed to base 28 by means of a ring-shaped weld, laminate, or adhesive section 40 surrounding, but spaced a short distance from aperture 38. Film 35 extends outwardly from section 40 and is affixed to the exterior edge of the bottom surface of flange 33.

This mounting structure increases the flexibility of label 26 to a small degree as compared to the construction where part 32 is affixed directly to the label surface. In addition, it permits part 32 to be displaced relative to the surface 29 of base 28, to a small extent, to permit the fingers of the user to be situated between label 26 and part 32 so as to absorb the force applied when part 20 is caused to frictionally engage part 32.

However, as indicated above, the structure disclosed in Figure 2 has two major drawbacks. When the fingers are inserted behind part 32, as shown in Figure 2, substantial stress is concentrated at points 42 near the exterior of section 40, adjacent the position of the fingers. These stress points 42 tend to disrupt the integrity of section 40 and may cause film member 35 to detach from base 28.

In addition, affixing film member 35 to the exterior edge of bottom surface of flange 33 creates a pocket 44 between the upper surface of film member 35 and lower surface of flange 33, into which waste material may collect. Because of the position and size of pocket 44, it is extremely difficult to remove the collected waste material therein. Consequently, the label may become unusable long before it would normally be necessary to remove the label from the skin.

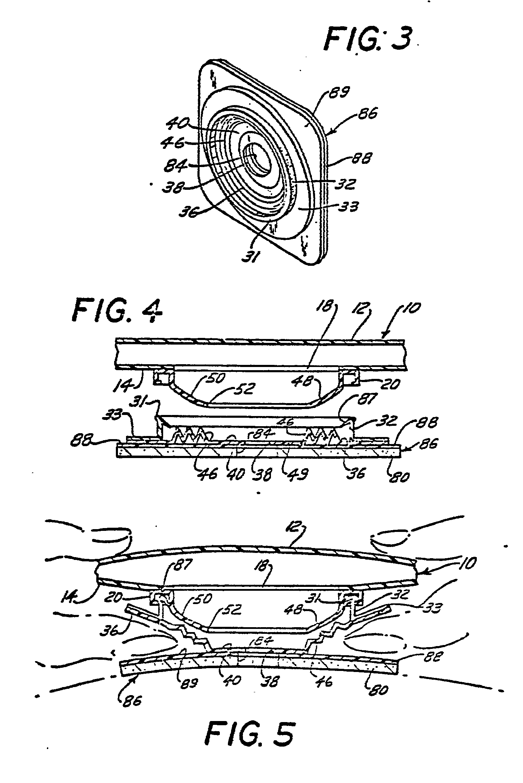

The latter disadvantage in particular is eliminated by the structure of the present invention. Figs. 3, 4 and 5 illustrate the structure and function of the first preferred embodiment of the present invention, which is designed to overcome the defects noted above. All parts of the device of the present invention illustrated in Figs. 3, 4 and 5 are identical to those illustrated in Figs. 1 and 2, with exceptions noted below. For this reason, a detailed description of the previously described parts and the functions of same is omitted.

Adhesive-backed label 86 comprises a base 88 which is preferably a thin film of polymeric material such as polyethylene and an adhesive layer 80 situated on the rear surface of base 88. Adhesive layer 80 is preferably formed as a homogeneous blend of one or more pressure-sensitive viscous or elastomeric materials having intermittently dispersed therein one or more water-soluble or swellable hydrocolloid gums and may also include one or more thermoplastic elastomers and/or one or more swellable cohesive strengthening agents.

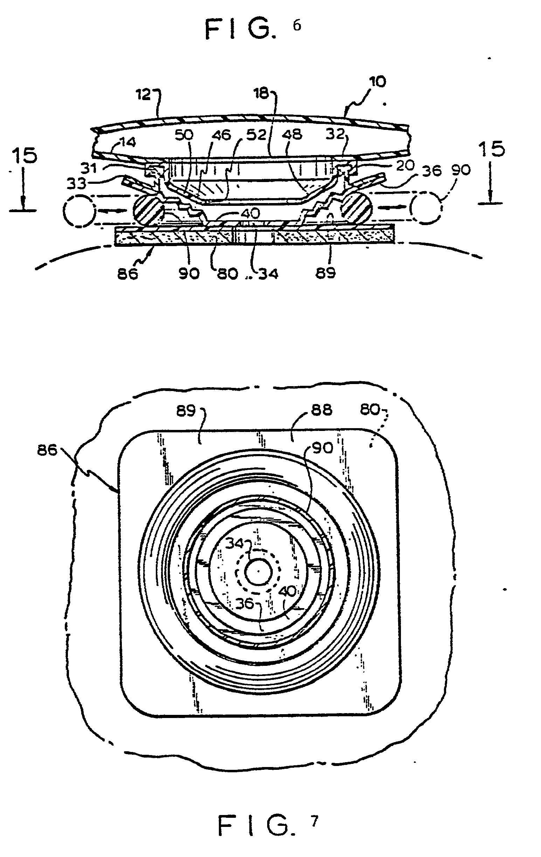

In the embodiments shown in Figures 3 to 5, and indeed in the embodiments of Figures 6 to 9 discussed hereinafter, a member 36 is employed to mount part 32 to base 88 of label 86, this member including an expandable means situated between welded section 40 and the portion of member 36, affixed to part 32 in the manner taught in the parent application, publication No. EP-A-0 098 718, from which the present application is divided, and which forms the subject matter of that parent application. Member 36 is preferably made of plastic or similar material of sufficient strength and thickness to maintain the pouch in a normal position proximate the surface of label 86, even when the pouch is filled with waste material. The structure of member 36, and particularly the expandable means, permits a greater degree of flexibility of the label, facilitates displacement of part 32 from its normal positions proximate the surface of label 86 during mounting of the pouch, and eliminates any stress points resulting from the displacment. In addition, by affixing member 36 to the bottom of part 32, adjacent the interior edge thereof, pocket 44 is eliminated and, as a result, the collection of waste material beneath part 32 is prevented.

Figure 3 illustrates the structure of the label. Figures 4 and 5 illustrate the manner in which the label facilitates engagement of parts 20 and 32 through the use of the expandable means which forms a portion of member 36. Figures 4 and 5 also illustrate the cover means associated with the part mounted on the pouch and the manner in which the cover means protects the expandable means when the parts are engaged, and which forms the subject matter of the present invention. The expandable means preferably comprises one or more accordion-like folds 46 (three are illustrated in Figures 3, 4 and 5) formed in member 36 at a location between section 40 and the portion of member 36 to which part 32 is affixed. Accordion-like folds 46 facilitate the displacement of part 32 relative to surface 89 by permitting member 36 to expand when the fingers are inserted between the part and the label, and, as a result, eliminate stress on points along section 40 such that the integrity of section 40 is not likely to be disrupted in normal usage.

It should be noted that, in the preferred embodiment of applicant's invention, member 36 is affixed to the bottom surface of flange 33 at a point spaced from accordion-like folds 46, through the use of an adhesive layer or the like. The upper surface of member 36 is adjacent the interior edge of part 32. This manner of affixation eliminates pocket 44 present in the conventional mounting structure between part 32 and member 36. Thus, with the structure of the present invention, it is not possible for any waste material to collect under part 32.

It should also be appreciated that flange 33, extending outwardly of protrusion 31, can be eliminated, if desired. In that case, member 36 would be affixed to the rear surface of protrusion 31, adjacent to the interior side thereof. This structure also eliminates the possibility of waste build-up under part 32.

According to the present invention part 20, affixed to wall 14 of pouch 10, is provided with a flange-like cover element 48 which is preferably mounted to and extends from the edge or side of part 20. Element 48 comprises a body portion 50 and rim 52. Body portion 50 is designed to be in registration with the accordion-like folds 46 on member 36, when parts 20 and 32 interengage. The purpose of body portion 50 is to cover and protect accordion-like folds 46 from waste material, which may move laterally along member 36 from the stoma receiving aperture towards part 32. The rim 52 of element 46 is biased, due to the resiliency of body 50, toward the section 49 of the surface of member 36, between folds 46 and aperture 38. In this manner, element 48 tends to seal the portion of member 36 containing folds 46 from any waste material.

It will be appreciated that accordion-like folds 46 may expand in a direction either parallel to or perpendicular to surface 89. The only limitation on the number and structure of the accordion-like folds is that member 36 must have sufficient strength to prevent substantial expansion during normal use of the device. That is, member 36 must have sufficient strength to withstand substantial expansion caused by the weight of the pouch, and thus maintain part 32 proximate surface 89, even when the pouch is filled to capacity.

Figures 6-9 illustrate the use of a means for retaining the expandable means 46 in the expanded condition. Retaining the folds 46 in the expanded condition will maintain the surface of the pouch at a location remote from the stoma, which may be required immediately after surgery.

The retaining means preferably comprises a ring-like element 190 composed of elastic material, such as closed cell foam or the like which can be expanded to fit over the outwardly extending flange 33 of part 32, and then released such that it lodges beneath member 36, preferably below part 32. Thus, element190 will then retain the folds 46 in the expanded condition.

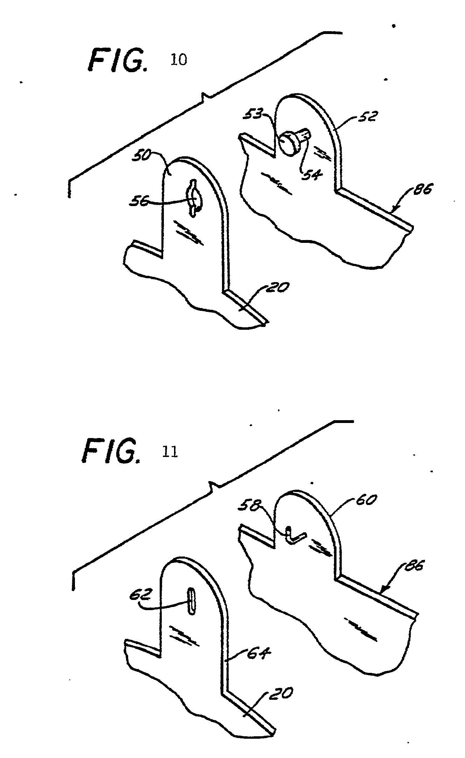

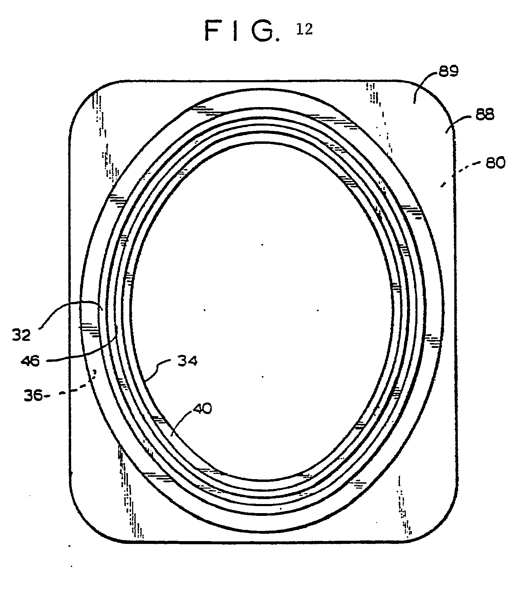

Element 190 can take various forms and may be detachable or simply pivotably connected to the label, by an articulated or flexible joint, in which case, it can be pivoted to a remote position when not in use. Moreover, element 190 may have a solid cross-section and be in the form of a detachable "O"-ring, as illustrated in Figs. 14 and 15, a detachable "O"-ring of hollow cross-section, as illustrated in Fig. 16 as 190', or be a detachable ring-like member 190" with an upstanding part 92 and an enlarged base 94, as illustrated in Fig. 17.

It should be understood that when an expansion retaining means is utilized, it is possible to convert the ostomy device into an irrigation and/or drain device by the addition of inlet and/or outlet ports in appropriate locations. This provides the device of the present invention with versatility not possible in conventional devices of this type.

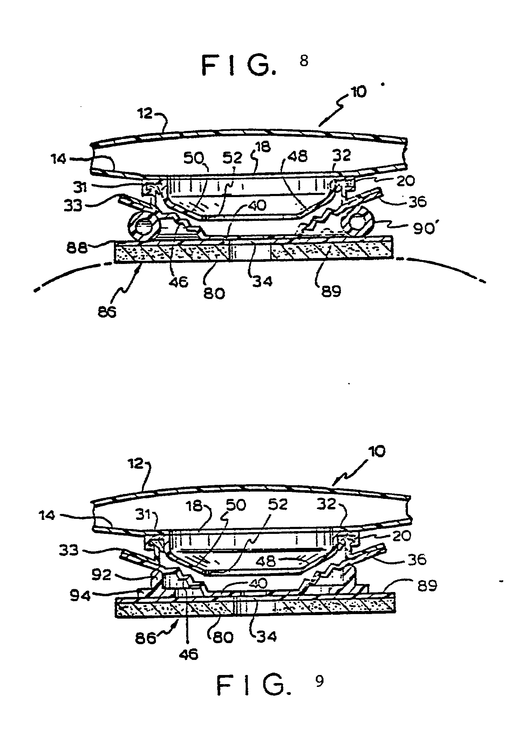

Figures 10 and 11, respectively, illustrate two preferred embodiments of another aspect of the present invention which relates to the use of auxiliary connecting means. Because the force which may be applied to cause frictional engagement of the interengaging parts is limited, it is difficult to prevent the occasional accidental detachment of the interengaging parts. For this reason, a device according to the present invention may be provided with auxiliary interengaging parts which engage independently of the primary interengaging parts and provide an extra measure of security against the accidental detachment of the pouch from the label. The auxiliary interengaging parts also aid to support the weight of the pouch when filled.

As illustrated in Figure 10, the auxiliary interengaging parts comprise a pair of aligned tabs 50, 52 extending from the top of pouch primary interengaging part 20 and label 86, respectively. Secondary interengaging part 52 is provided with a protrusion 54 extending from one surface thereof. protrusion 54 is preferably provided with an enlarged tip 53. The other part 50 is provided with an aperture 56 designed to frictionally receive protrusion 54 therein. Before or after the primary interengaging parts 20 and 32 are frictionally engaged, the user places her fingers on either side of the aligned tab pair 50, 52 and forces the protrusion 54 into the aperture 56, such that enlarged tip 53 is situated adjacent the opposite surface of tab 50, so as to releasably connect the tabs. When the pouch is removed from the label, the process is reversed, with protrusion 54 being pushed out from the aperture 56 to release the tabs.

Figure 11 shows a modification of this aspect of the present invention wherein the protrusion and aperture combination is replaced with a hook and eye configuration. In this case, hook 58 extends from one tab 60 and is designed to engage aperture 62 on the other tab 64 when primary interengaging parts 20 and 32 are connected.

Whichever version of the secondary interengaging parts is utilized, it is preferable that the tabs extend upwardly from part 20 and label 86 at the tops thereof. In this position, the secondary interengaging parts will retain the pouch on the label, even if parts 20 and 32 become disconnected.

Figure 12 illustrates a modification of the shape of the interengaging parts 20 and 32 where the parts are elongated, preferably oval, as opposed to circular, as illustrated in Figure 10. This configuration permits the use of the device with elongated wounds and after certain types of surgery which requires an unobstructed elongated area around the wound.

It will now be appreciated that the present invention relates to an ostomy device with a label and a detachable pouch wherein the pocket or gap between the mounting member and the interengaging part is eliminated such that the accumulation of waste material beneath the interengaging part is prevented.

Accumulation of waste material beneath the interengaging part mounted to the label is prevented by affixing the under surface of the part to the mounting member adjacent the interior edge of the part. Thus, no space between the part and member is present.

Accidental detachment of the pouch from the label is prevented by providing secondary connecting means in the form of auxiliary interengaging parts. The auxiliary interengaging parts preferably extend from the top of the pouch primary interengaging part and label, and preferably take the form of interlocking tabs including a protrusion and aperture or hook and eye configuration.

Through the use of expansion retaining means, the expansion of the accordion-like fold can be maintained in order to keep the pouch at a location remote from the stoma. Moreover, for wounds which require an unobstructed elongated area, the interengaging parts may be made oval instead of round.

While only a limited number of preferred embodiments of the present invention have been disclosed herein for purposes of illustration, it is obvious that many variations and modifications could be made thereto.

## Claims

1. An ostomy device comprising a pouch (10) having a stoma receiving aperture (18), a separate adhesively-backed label (26, 86) for attachment of the device to the skin of the wearer and having an aperture (34) coaxially aligned with the aperture (18) in the pouch for the passage of the stoma through the label, and annular mounting means for releasably connecting the pouch to the label, wherein the annular mounting means comprise releasably interengaging first and second annular parts (20, 32) each provided with releasably engaging interconnecting means, and means for mounting said first and second parts respectively on the pouch (10) and the label (26, 86) around their respective apertures (18, 34), characterised in that the first annular part (20) of the mounting means connected to the pouch (10) has a cover (48, 50) having a central aperture therein aligned with the aperture (18) in the pouch and the aperture (34) in the label, said rover (48, 50) extending inwardly from the periphery of said part (20) and axially towards said second part (32), and having a rim (52), which, when the two interconnecting parts (20, 32) are engaged, sealingly engages with the surface of the label (26, 86) interiorly of said second part and around the aperture (34).

2. A device according to claim 1, wherein the second part (32) of the mounting means comprises an annular member (32) having means (31) for releasably engaging with cooperating means on the first part, and wherein the label (86) comprises a flexible backing member (88) coated with a layer of adhesive (80), said annular member (32) being mounted on the label (86) by a flexible member (36) connected to and extending inward of the annular member (32) and connected to the backing member (88) adjacent the aperture (84) therein, said flexible member permitting the annular member (32) to be drawn away from the label (86) whilst the connection is made between the two interengaging parts (20, 32).

3. A device according to claim 2, wherein the annular member (32) of the mounting means connected to the label (86) comprises an integral flange (33) extending radially outward from that end of the annular member (32) which extends towards said label, and away from the pouch, and wherein the flexible member (36) is connected to and extends radially inward from said flange (33).

4. A device according to claim 2 or 3, wherein the flexible member (36) is sealed to the upper surface (80) of the backing member (88) around the periphery of the aperture (34) therein and presents a flat surface (40) which, when the two interengaging parts (20, 32) are engaged, is sealingly contacted by the rim (52) of said cover (48, 50).

5. A device according to any one of claims 1–4, wherein one of interengageable parts (20, 32) comprises an undercut annular channel member, and the other an annular protrusion (31) which is a frictional fit in said channel member.

6. A device according to any one of the preceding claims, wherein said first part comprises a ring-shaped engaging part (20) and wherein said rover means comprises a flange-like element (50) extending radially inward from said engaging part.

7. A device according to any one of claims 2–6 in combination with means (190) for retaining said second part (32) in an extended position, wherein the said second part is displaced away from the surface of the label (86).

8. A device according to claim 7, wherein said retaining means comprises an element (190) interposable between said second part mounting means and said label.

9. A device according to claim 7 or 8, wherein said retaining means is detachable.

10. A device according to claim 7, 8 or 9, wherein said retaining means comprises a ring-like element.

11. A device according to any one of claims 7–10, wherein said retaining means (190) is composed of elastic material.

12. A device according to any one of claims 7–10, wherein said retaining means (190) is composed of closed cell foam.

13. A device according to any one of claims 7–12, wherein said retaining means (190) has a substantially circular cross-section.

14. A device according to any one of claims 7–12, wherein said retaining means (190) comprises an upstanding section (92) and a base section (94).

15. A device according to any one of claims 1–14, wherein said interengaging parts (20, 32) have an elongated configuration.

16. A device according to claim 15, wherein said interengaging parts (20, 32) have a substantially oval configuration.

## Patentansprüche

1. Ostomievorrichtung mit einem Beutel (10), der eine Öffnung (18) zur Aufnahme eines Stomas aufweist, einem getrennten auf der Rückseite mit Klebstoff versehenen Schild (26, 86) zum Anbringen der Vorrichtung an der Haut des Benutzers und das eine Öffnung (34) aufweist, die koaxial mit der Öffnung (18) in dem Beutel ausgerichtet ist für den Durchgang des Stomas durch das Schild, und eine ringförmige Befestigungseinrichtung zum lösbaren Verbinden des Beutels an dem Schild, wobei die ringförmige Befestigungseinrichtung lösbar miteinander in Eingriff bringbare erste und zweite ringförmige Teile (20, 32) aufweisen, die jeweils mit lösbar in Eingriff bringbaren Verbindungseinrichtungen versehen sind, und Einrichtungen zum Befestigen des ersten bzw. zweiten Teils an dem Beutel (10) bzw. dem Schild (26, 86) um die jeweiligen Öffnungen (18, 34), dadurch gekennzeichnet, daß das erste ringförmige Teil (20) der Befestigungseinrichtung, die mit dem Beutel (10) verbunden ist, eine Abdeckung (48, 50) mit einer zentralen Öffnung darin hat, die mit der Öffnung (18) in dem Beutel und der Öffnung (34) in dem Schild ausgerichtet ist, wobei die Abdeckung (48, 50) sich von dem Umfang des Teils (20) nach innen zu dem zweiten Teil (32) axial erstreckt, und mit einem Rand (52), der, wenn die zwei Verbindungsteile (20, 32) in Eingriff sind, abdichtend mit der Fläche des Schildes (26, 86) im Innern des zweiten Teils und um die Öffnung (34) in Eingriff steht.

2. Vorrichtung nach Anspruch 1, wobei das zweite Teil (32) der Befestigungseinrichtung ein ringförmiges Element (32) aufweist, mit Einrichtungen (31) zum lösbaren Ineingriffbringen mit zusammenwirkenden Einrichtungen an dem ersten Teil, und wobei das Schild (86) ein flexibles Halteelement (88) aufweist, das mit einer Klebstoffschicht (80) beschichtet ist, das ringförmige Element (32) an dem Schild (86) befestigt ist mittels eines flexiblen Elements (36), das mit dem ringförmigen Element (32) verbunden ist und sich innerhalb zu diesem erstreckt und mit dem Halteelement (88) benachbart zu der darin angeordneten Öffnung (84) verbunden ist; das flexible Element ermöglicht, daß das ringförmige Element (32) von dem Schild (86) abgezogen werden kann, während die Verbindung zwischen den zwei miteinander in Eingriff stehenden Teilen (20, 32) hergestellt ist.

3. Vorrichtung nach Anspruch 2, wobei das ringförmige Element (32) der Befestigungseinrichtung, die mit dem Schild (86) verbunden ist, einen einstückigen Flansch (33) aufweist, der sich radial nach außen erstreckt, von dem Ende des ringförmigen Elements (32), das sich in Richtung zu dem Schild erstreckt, und weg von dem Beutel und wobei das flexible Element (36) verbunden ist mit dem Flansch (33), und sich radial von diesem nach innen erstreckt.

4. Vorrichtung nach Anspruch 2 oder 3, wobei das flexible Element (36) mit der oberen Fläche (80) des Halteelements (88) abgedichtet ist, entlang des Umfangs der darin befindlichen Öffnung (34) und eine flache Fläche (40) darstellt, die, wenn die zwei miteinander in Eingriff stehenden Teile (20, 32) in Eingriff sind, abdichtend von dem Rand (52) der Abdeckung (48, 50) berührt wird.

5. Vorrichtung nach einem der Ansprüche 1 bis 4, wobei eines der miteinander in Eingriff bringbaren Teile (20, 32) ein ringförmiges eingeschnittenes Kanalelement aufweist und das andere einen ringförmigen Vorsprung (31) aufweist, das einen Reibungssitz in dem Kanalelement bildet.

6. Vorrichtung nach einem der vorstehenden Ansprüche, wobei das erste Teil ein ringförmiges Aufnahmeteil (20) aufweist und wobei das Abdeckelement ein flanschartiges Element (50) aufweist, das sich radial von dem Aufnahmeelement nach innen erstreckt.

7. Vorrichtung nach einem der Ansprüche 2 bis 6 in Kombination mit Einrichtungen (190) zum Festhalten des zweiten Teils (32) in einer verlängerten Position, wobei das zweite Teil von der Fläche des Schildes (86) entfernt gehalten wird.

8. Vorrichtung nach Anspruch 7, wobei die Halteeinrichtung ein Element (190) aufweist, das zwischen dem zweiten Teil der Befestigungseinrichtung und dem Schild einsetzbar ist.

9. Vorrichtung nach Anspruch 7 oder 8, wobei die Halteeinrichtung abnehmbar ist.

10. Vorrichtung nach Anspruch 7, 8 oder 9, wobei die Halteeinrichtung ein ringförmiges Element aufweist.

11. Vorrichtung nach einem der Ansprüche 7 bis 10, wobei die Halteeinrichtung (190) aus elastischem Material gebildet ist.

12. Vorrichtung nach einem der Ansprüche 7 bis 10, wobei die Halteeinrichtung (190) aus einem Schaum mit geschlossenen Zellen gebildet ist.

13. Vorrichtung nach einem der Ansprüche 7 bis 12, wobei die Halteeinrichtung (190) einen im wesentlichen kreisförmigen Querschnitt hat.

14. Vorrichtung nach einem der Ansprüche 7 bis 12, wobei die Halteeinrichtung (190) einen aufrechtstehenden Abschnitt (92) und einen Basisabschnitt (94) aufweist.

15. Vorrichtung nach einem der Ansprüche 1 bis 14, wobei die miteinander in Eingriff bringbaren Teile (20, 32) eine längsgestreckte Ausbildung haben.

16. Vorrichtung nach Anspruch 15, wobei die miteinander in Eingriff bringbaren Teile (20, 32) eine im wesentlichen ovale Ausbildung haben.

**Revendications**

1. Dispositif de stomie comprenant une poche (10) percée d'une ouverture (18) de réception d'anus artificiel, une étiquette (26, 86) à dos adhésif séparée permettant de fixer le dispositif à la peau de l'utilisateur et comportant une ouverture (34) coaxiale à l'ouverture (18) de la poche pour permettre le passage de l'anus artificiel à travers l'étiquette, et des moyens de montage annulaires permettant de raccorder de manière amovible la poche à l'étiquette, dans lequel les moyens de montage annulaires comprennent une première et une seconde pièces annulaires (20, 32) à prise mutuelle amovible munies chacune de moyens d'interconnexion à prise amovible, et des moyens permettant de monter lesdites première et seconde pièces respectivement sur la poche (10) et sur l'étiquette (26, 86) autour de leurs ouvertures respectives (18, 34), caractérisé en ce que la première pièce annulaire (20) des moyens de montage raccordée à la poche (10) comporte une couverture (48, 50) percée d'une ouverture centrale en regard de l'ouverture (18) de la poche et de l'ouverture (34) de l'étiquette, ladite couverture (48, 50) s'étendant vers l'intérieur à partir du pourtour de ladite pièce (20) et axialement en direction de ladite seconde pièce (32), et comportant un rebord (52), qui, quand les deux pièces (20, 32) à prise mutuelle sont en prise, s'engage de façon étanche sur la surface de l'étiquette (26, 86), intérieurement à ladite seconde pièce et autour de l'ouverture (34).

2. Dispositif selon la revendication 1, dans lequel la seconde pièce (32) des moyens de montage comprend un élément annulaire (32) comportant un moyen (31) destiné à venir en prise amovible avec des moyens coopérants sur la première pièce, et dans lequel l'étiquette (86) comprend un élément dorsal souple (88) revêtu d'une couche d'adhésif (80), ledit élément annulaire (32) étant monté sur l'étiquette (86) par un élément souple (36) qui est raccordé à et s'étend vers l'intérieur de l'élément annulaire (32) et qui est raccordé à l'élément dorsal (88) à proximité de l'ouverture (84) de celui-ci, ledit élément souple permettant de retirer l'élément annulaire (32) de l'étiquette (86) pendant que l'on réalise le raccordement entre les deux pièces à prise mutuelle (20, 32).

3. Dispositif selon la revendication 2, dans lequel l'élément annulaire (32) des moyens de montage raccordés à l'étiquette (86) comprend une bride intégrale (33) s'étendant radialement vers l'extérieur à partir de l'extrémité de l'élément annulaire (32) qui s'étend vers ladite étiquette, et à l'opposé de la poche, et dans lequel l'élément souple (36) est raccordé à et s'étend radialement vers l'interieur à partir de ladite bride (33).

4. Dispositif selon la revendication 2 ou 3, dans lequel l'élément souple (36) est soudé à la surface supérieure (80) de l'élément dorsal (88) autour du pourtour de l'ouverture (34) de celui-ci et présente une surface plate (40) qui, quand les deux pièces à prise mutuelle (20, 32) sont en prise, est en contact étanche avec le rebord (52) de ladite couverture (48, 50).

5. Dispositif selon l'une quelconque des revendications 1 à 4, dans lequel l'une des pièces (20, 32) à prise mutuelle comprend un élément découpé en forme de canal annulaire, et l'autre une saillie annulaire (31) qui s'ajuste par frottement dans ledit élément en forme de canal.

6. Dispositif selon i'une quelconque des revendications précédentes, dans lequel ladite première pièce comprend une pièce de prise en forme d'anneau (20) et dans lequel ledit couvercle comprend un élément en forme de bride (50) s'étendant radialement vers l'intérieur à partir de ladite pièce de prise.

7. Dispositif selon l'une quelconque des revendications 2 à 6, en combinaison avec un moyen (90) servant à maintenir ladite seconde pièce (32) en extension, dans lequel ladite seconde pièce est maintenue à distance de la surface de l'étiquette (86).

8. Dispositif selon la revendication 7, dans lequel ledit moyen de maintien comprend un élément (90) interposable entre ledit moyen de montage de la seconde pièce et ladite étiquette.

9. Dispositif selon la revendication 7 ou 8, dans lequel ledit moyen de maintien est détachable.

10. Dispositif selon la revendication 7, 8 ou 9, dans lequel ledit moyen de maintien comprend un élément en forme d'anneau.

11. Dispositif selon l'une quelconque des revendications 7 à 10, dans lequel ledit moyen de maintien (90) est composé d'une matière élastique.

12. Dispositif selon l'une quelconque des revendications 7 à 10, dans lequel ledit moyen de maintien (90) se compose d'une mousse à cellules fermées.

13. Dispositif selon l'une quelconque des revendications 7 à 12, dans lequel ledit moyen de maintien (90) a une section droite de forme sensiblement circulaire.

14. Dispositif selon l'une quelconque des revendications 7 à 12, dans lequel ledit moyen de maintien (90) comprend une partie verticale (92) et une base (94).

15. Dispositif selon l'une quelconque des revendications 1 à 14, dans lequel lesdites pièces à prise mutuelle (20, 32) sont de forme allongée.

16. Dispositif selon la revendication 15, dans lequel lesdites pièces à prise mutuelle (20, 32) sont de forme sensiblement ovale.

FIG. 1

FIG. 2

FIG. 3

FIG. 4

FIG. 5

FIG. 6

FIG. 7

# F I G. 8

# F I G. 9

**FIG.** 10

**FIG.** 11

F I G. 12